# EUROPÄISCHE PATENTSCHRIFT

(11) **EP 0 740 658 B1**
(45) Veröffentlichungstag und Bekanntmachung des Hinweises auf die Patenterteilung: **02.07.2003**
(21) Anmeldenummer: 95901436.6
(22) Anmeldetag: 26.11.1994
(51) Int. Cl.: C07D 209/52, A61K 31/40, C07D 409/04, C07D 209/90, C07D 403/06, C07D 409/06, C07D 405/06, C07D 403/04, C07D 413/06, C07D 209/46, C07D 209/96

(54) **N-SUBSTITUIERTE AZABICYCLOHEPTAN-DERIVATE UND IHRE VERWENDUNG, Z.B., ALS NEUROLEPTIKA**
N-SUBSTITUTED AZABICYCLOHEPTANE DERIVATIVES USED, FOR EXAMPLE, AS NEUROLEPTICS
DERIVES N-SUBSTITUES D'AZABICYCLOHEPTANE UTILISES, PAR EXEMPLE, COMME NEUROLEPTIQUES

(30) Priorität: 04.12.1993 DE 4341402
(43) Veröffentlichungstag der Anmeldung: 06.11.1996
(73) Patentinhaber: Abbott GmbH & Co. KG, 65205 Wiesbaden (DE)
(72) Erfinder: STEINER, Gerd, D-67281 Kirchheim (DE); MUNSCHAUER, Rainer, D-67434 Neustadt (DE); UNGER, Liliane, D-67065 Ludwigshafen (DE); TESCHENDORF, Hans-Jürgen, D-67373 Dudenhofen (DE); HÖGER, Thomas, D-68535 Edingen-Neckarhausen (DE)
(74) Vertreter: Grünecker, Kinkeldey, Stockmair & Schwanhäusser Anwaltssozietät
(86) Internationale Anmeldenummer: EP9403910
(87) Internationale Veröffentlichungsnummer: WO95015312

(56) Entgegenhaltungen:
- WO-A-92/18480
- WO-A-94/00458
- DE-A- 4 219 973

## Beschreibung

Die Erfindung betrifft N-substituierte Azabicycloheptan-Derivate, deren Herstellung und Verwendung zur Herstellung von Pharmaka.

Es ist bekannt, daß basisch substituierte Butyrophenon-Derivate bzw. Benzoesäureamid-Derivate Wirkungen als Neuroleptika bzw. Gehirnprotektiva aufweisen (US 4 605 655, EP 410 114, DE 12 89 845, EP 400 661, DE 29 41 880, EP 190 472, DE 42 19 973, WO 92/18480, WO 94/00458).

Hierbei scheinen die beobachteten Affinitäten zu Dopamin- und Serotonin-Rezeptorsubtypen eine besondere Rolle zu spielen.

Es wurde nun gefunden, daß N-substituierte 3-Azabicyclo[3.2.0]-heptan-Derivate der Formel I 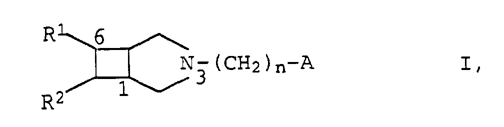 worin
- R¹: eine gegebenenfalls durch Halogenatome, C₁-C₄-Alkyl, Trifluormethyl-, Hydroxy-, C₁-C₄-Alkoxy-, Amino-, Monomethylamino-, Dimethylamino-, Cyano- oder Nitrogruppen mono- oder disubstituierte Phenyl- oder Thienylgruppe bedeutet,
- R²: ein Wasserstoffatom oder eine gegebenenfalls durch Halogen, Methoxy, Hydroxy oder Amino substituierte Phenylgruppe ist,
n die Zahl 1, 2, 3 oder 4 bedeutet,
A ein Wasserstoffatom oder einer der Reste 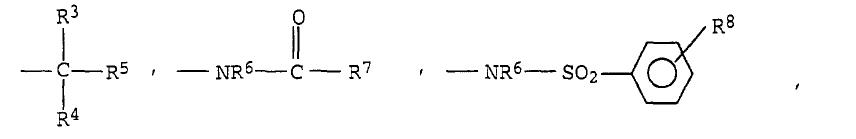 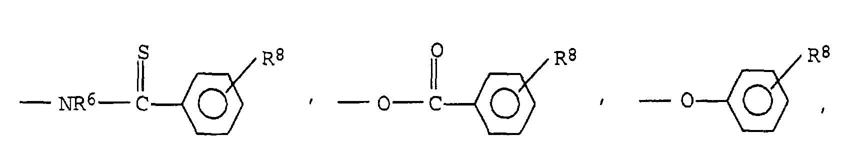 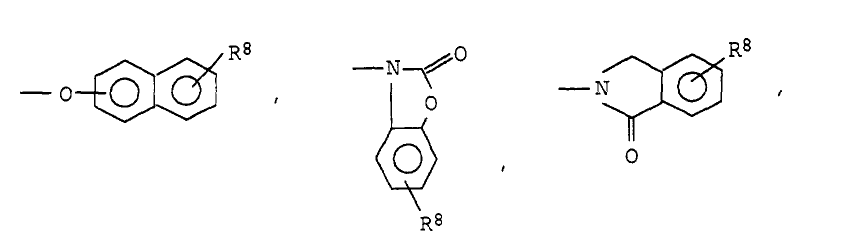 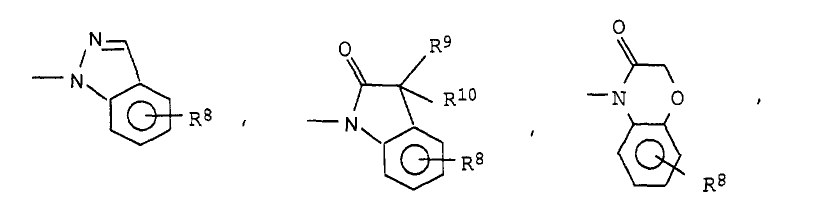 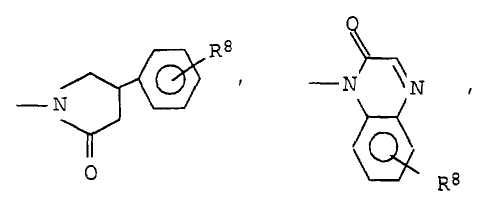 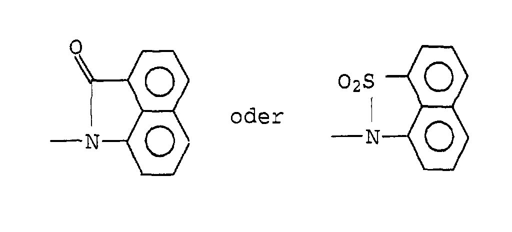 ist,
- R³: ein Wasserstoffatom oder einen Hydroxyrest darstellt,
- R⁴: ein Wasserstoffatom bedeutet oder
- R³ und R⁴: zusammen ein Sauerstoffatom darstellen,
- R⁵: eine gegebenenfalls durch Fluor oder Chlor substituierte Thienyl- oder Naphthylgruppe bedeutet,
- R⁶: ein Wasserstoffatom oder eine Methylgruppe darstellt,
- R⁷: eine durch Amino, C₁₋₄-Alkylamino oder Di-C₁₋₄-alkylamino monosubstituierte Phenylgruppe oder eine gegebenenfalls durch Fluor, Chlor oder Nitro substituierte Thienyl-, Naphthyl-, Benzofuryl-, Benzothienyl-, Indolyl-, N-Methylindolyl- oder Indenylgruppe oder eine C₃- bis C₆-Cycloalkylgruppe bedeutet,
- R⁸: Wasserstoff, Fluor, Chlor, C₁₋₄-Alkyl, Methoxy oder Amino bedeutet,
- R⁹: Wasserstoff oder eine Methylgruppe darstellt und
- R¹⁰: Wasserstoff oder eine Methylgruppe darstellt oder
- R⁹ und R¹⁰: zusammen mit dem Ring-C-Atom einen Spirocyclopropanring darstellen,
und deren Salze mit physiologisch verträglichen Säuren wertvolle pharmakologische Eigenschaften besitzen.

In der Formel I haben die Substituenten R¹ bis R¹⁰ sowie n vorzugsweise folgende Bedeutungen:
- R¹:: Phenyl und Thienyl, gegebenenfalls durch Fluor, Chlor, Jod, Methoxy, Trifluormethyl oder Nitro substituiert
- R²:: Wasserstoff
- n:: 1 und 2
- R³:: Wasserstoff
- R⁴:: Wasserstoff
- R⁵:: 1-Naphthyl
- R⁶:: Wasserstoff
- R⁷:: o-Aminophenyl, o-N-Methylaminophenyl, 5-Chlor-thien-1-yl, 1-Naphthyl, 3-Indenyl, Cyclohexyl, 3-Chlor-l-benzothien-2-yl
- R⁸:: Wasserstoff
- R⁹:: Wasserstoff, Methyl
- R¹⁰:: Wasserstoff, Methyl

Die erfindungsgemaßen Verbindungen der Formel I lassen sich herstellen, indem man eine Verbindung der Formel II

Nu-(CH₂)ₙ-A (II)

in der A und n die angegebenen Bedeutungen haben und Nu eine nukleofuge Abgangsgruppe darstellt, mit einem 3-Azabicyclo[3.2.0]heptan Derivat der Formel III 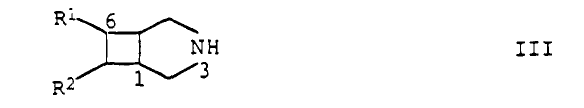 worin
- R¹: eine gegebenenfalls durch Halogenatome, C₁-C₄-Alkyl-, Trifluormethyl, Hydroxy-, C₁-C₄-Alkoxy-, Amino-, Monomethylamino-, Dimethylamino-, Cyano- oder Nitrogruppen mono- oder disubstituierte Phenyl- oder Thienylgruppe bedeutet und
- R²: ein Wasserstoffatom oder eine gegebenenfalls durch Halogen, Methoxy, Hydroxy oder Amino substituierte Phenylgruppe ist,
umsetzt und die so erhaltenen Verbindungen gegebenenfalls in ihre Säureadditionssalze mit physiologisch verträglichen Säuren überführt.

Als nukleofuge Abgangsgruppe für Nu kommen vorzugsweise Halogenatome, insbesondere Brom oder Chlor, in Betracht.

Die Umsetzung erfolgt zweckmaßig in Gegenwart einer inerten Base, wie Triethylamin oder Kaliumcarbonat, als saurebindendes Mittel in einem inerten Lösungsmittel, wie einem cyclischen gesättigten Ether, insbesondere Tetrahydrofuran oder Dioxan, oder einem Benzolkohlenwasserstoff, wie Toluol oder Xylol.

Die Umsetzung erfolgt in der Regel bei Temperaturen von 20 bis 150°C, und ist im allgemeinen innerhalb von 1 bis 10 Stunden beendet.

Die erfindungsgemäßen Verbindungen der Formel I können entweder durch Umkristallisation aus den üblichen organischen Lösungsmitteln, bevorzugt aus einem niederen Alkohol, wie Ethanol, umkristallisiert oder durch Säulenchromatographie gereinigt werden.

Racemate lassen sich in einfacher Weise durch klassische Spaltung mit optisch aktiven Carbonsauren, z.B. Weinsäure-Derivaten, in einem inerten Losungsmittel, z.B. niederen Alkoholen, in die Enantiomeren auftrennen.

Die freien 3-Azabicyclo[3.2.0]heptan-Derivate der Formel I konnen in ublicher weise in das Säureadditionssalz einer pharmakologisch vertraglichen Saure uberfuhrt werden, vorzugsweise durch Versetzen einer Lösung mit einem Äquivalent der entsprechenden Säure. Pharmazeutisch verträgliche Sauren sind beispielsweise Salzsaure, Phosphorsaure, Schwefelsäure, Methansulfonsäure, Amidosulfonsaure, Maleinsäure, Fumarsäure, Oxalsäure, Weinsaure oder Zitronensäure.

Die erfindungsgemaßen Verbindungen weisen wertvolle pharmakologische Eigenschaften auf. Sie können als Neuroleptika (insbesondere atypische), Antidepressiva, Sedative, Hypnotika, ZNS-Protektiva oder Muskelrelaxantien Verwendung finden. Mehrere der genannten Wirkungsqualitaten können kombiniert bei einer erfindungsgemäßen Verbindung auftreten. Der pharmakologische Wirkungsnachweis erfolgt sowohl in vivo wie auch in vitro, wobei die Substanzcharakterisierung insbesondere durch die teilweise sehr hohe und selektive Affinität zu Rezeptor-Subtypen, z.B. Dopamin D₁-, D₂-, D₃- und vor allem D₄-Rezeptoren; Serotonin 1A-, 1D- und 2-Rezeptoren; Alpha 1- und 2-Rezeptoren; Histamin 1- sowie Muscarin-Rezeptoren, möglich ist.

Für die in vivo Charakterisierung der neuen Substanzen wurden folgende Methoden herangezogen:
a) Beeinflussung der Orientierungsmotilität
   Mäuse zeigen in einer neuen Umgebung ein vermehrtes Explorationsverhalten, das sich in einer gesteigerten motorischen Aktivität äußert. Diese motorische Aktivität wird in Lichtschrankenkäfigen für die Zeit von 0 - 30 min nach Einsetzen der Tiere (NMRI-Mäuse, weibl.) in die Käfige gemessen. ED50: Dosis, die die motorische Aktivität im Vergleich zu Placebo-behandelten Kontrollen um 50 % reduziert.
b) Apomorphin-Antagonismus
   Weibliche NMRI-Mause erhalten 1,21 mg/kg Apomorphin s.c. Apomorphin führt in dieser Dosis zu einer motorischen Aktivierung, die sich, wenn man die Tiere in Maschendrahtkafigen halt, in einem permanenten Klettern äußert. Das Klettern wird mit einem Score bewertet (alle 2 min wahrend 30 min):
   0: Tier hat vier Pfoten am Boden
   1: Tier hat zwei Pfoten am Draht
   2: Tier hat vier Pfoten am Draht (klettert).
   Durch Vorbehandlung mit Antipsychotika ist das Kletterverhalten zu hemmen.
   ED50: Dosis, die die Kletteraktivitat der Tiere im Vergleich zu Placebo-behandelten Kontrollen um 50 % hemmt.
c) L-5-HTP-Antagonismus
   Weibliche Sprague-Dawley-Ratten erhalten L-5-HTP in einer Dosis von 316 mg/kg i.p. Die Tiere entwickeln darauf ein Erregungssyndrom, von dem Symptome
   - for paw treading und
   - tremor
   mit Hilfe eines Scores (O = nicht vorhanden, 1 = mäßig, 2 = deutlich ausgeprägt) alle 10 min in der Zeit von 20 bis 60 min nach L-5-HTP-Gabe bewertet werden. Im Mittel wird nach L-5-HTP-Gabe ein Score von 17 erreicht. Die Prüfsubstanzen p.o. werden 60 min vor L-5-HTP gegeben. Als ED50 wird die Dosis errechnet, die im Mittel den Kontrollscore um 50 % vermindert.

Die aufgeführten Methoden sind geeignet, Substanzen als Antipsychotika zu charakterisieren. Mit der Hemmung des L-5-HTP-Syndroms kann eine Serotonin-antagonistische Wirkung aufgezeigt werden, eine Wirkungsqualitat, wie sie für die sogenannten atypischen Neuroleptika charakteristisch ist.

Die erfindungsgemäßen Substanzen zeigen in diesen Tests eine gute Wirkung.

Die Erfindung betrifft dementsprechend auch ein therapeutisches Mittel, gekennzeichnet durch einen Gehalt an einer Verbindung der Formel I oder deren pharmakologisch verträgliches Säureadditionssalz als Wirkstoff neben üblichen Trager- und Verdünnungsmitteln, sowie die Verwendung der neuen Verbindungen bei der Bekämpfung von Krankheiten.

Die erfindungsgemäßen Verbindungen konnen in üblicher Weise oral oder parenteral, intravenös oder intramuskular verabfolgt werden.

Die Dosierung hangt vom Alter, Zustand und Gewicht des Patienten sowie von der Applikationsart ab. In der Regel betragt die tagliche Wirkstoffdosis zwischen etwa 1 und 100 mg/kg Korpergewicht bei oraler Gabe und zwischen 0,1 und 10 mg/kg Korpergewicht bei parenteraler Gabe.

Die neuen Verbindungen können in gebräuchlichen galenischen Applikationsformen fest oder flüssig angewendet werden, z.B. als Tabletten, Filmtabletten, Kapseln, Pulver, Granulate, Dragees, Suppositorien, Losungen, Salben, Cremes oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al: Pharmazeutische Technologie, Thieme-Verlag, Stuttgart, 1978). Die so erhaltenen Applikationsformen enthalten den Wirkstoff normalerweise in einer Menge von 1 bis 99 Gew.-%.

Die als Ausgangsstoffe für die Synthese der neuen Verbindungen benötigten Substanzen der Formel II sind bekannt oder lassen sich nach literaturbekannten Methoden herstellen.

Die Substanzen der Formel III lassen sich herstellen, indem man ein Amin der Formel IV 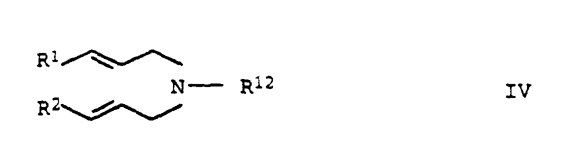 worin R¹ und R² die oben angegebenen Bedeutungen besitzen und R¹² Wasserstoff, Acetyl, Benzyl oder Trifluoracetyl bedeutet, photochemisch einer 2+2 Cycloaddition unterwirft und anschließend gegebenenfalls eine Acyl- oder Benzylgruppe abspaltet.

Die Photoreaktion gelingt gut in einem inerten Lösungsmittel, vorzugsweise Aceton, bei Temperaturen von 20 bis 80°C. Als Lichtquelle eignet sich besonders gut eine Quecksilberhochdrucklampe. Es ist gegebenenfalls vorteilhaft, die Photocycloaddition in einer Quarzapparatur unter einer Stickstoffatmosphare gegebenenfalls unter Zusatz von etwa 1 Mol Salzsaure pro Mol Amin durchzufuhren.

Die Photocycloaddition verlauft in den meisten Fällen hochdiastereoselektiv zu den bicyclischen Verbindungen III mit der exo-Konfiguration bezuglich R¹ und R²:

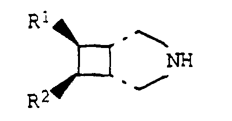

Durch Racematspaltung, z.B. mit optisch aktiven Weinsaure-Derivaten, lassen sich die beiden Enantiomeren rein isolieren.

Die Abspaltung eines Acylrestes (R¹²) erfolgt zweckmäßig durch verseifung nach bekannten Methoden. Analoges gilt für die Abspaltung des Benzylrestes.

Die Amine der Formel IV sind literaturbekannt oder lassen sich herstellen, indem man entweder einen Aldehyd R¹-CHO mit Vinylmagnesiumchlorid zum Allylalkohol V 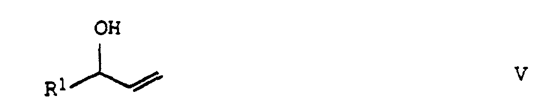 umsetzt, anschließend mit Chlorwasserstoff zum Allylchlorid VI 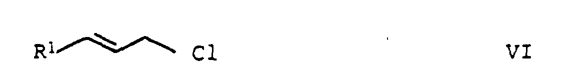 umlagert und zuletzt mit dem entsprechenden Allylamin VII 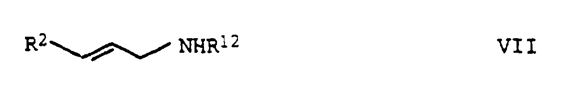 umsetzt, oder man unterzieht einen Zimtaldehyd VIII 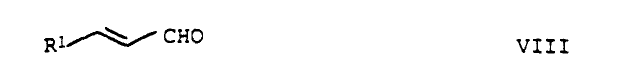 direkt der reduktiven Aminierung mit dem Allylamin VII.

Die folgenden Beispiele dienen zur Erläuterung der Erfindung:
A) Herstellung der Ausgangsmaterialien
   1. exo-6-(p-Fluor)-phenyl-3-azabicyclo[3.2.0]heptan
      19,4 g (102 mM) N-Allyl-N-[3-(4-fluorphenyl)allyl]amin in 130 ml Aceton wurden mit 130 ml 10 %iger Salzsäure sowie mit 600 mg Michlers Keton versetzt und unter Stickstoff 55 h mit einer 150 Watt Quecksilber-Hochdrucklampe in einer Quarzapparatur bei Raumtemperatur bestrahlt. Anschließend engte man den Reaktionsansatz ein und verteilte den Rückstand zwischen Methylenchlorid und Wasser. Man stellte mit wäßriger Ammoniaklösung alkalisch und extrahierte die wäßrige Phase noch zweimal mit Methylenchlorid nach. Die vereinigten organischen Phasen wurden mit Natriumsulfat getrocknet und eingeengt.
      Ausbeute 19,3 g (99 %), Schmp. 165-166°C (Maleinat).
      Zur Trennung der Antipoden versetzte man 15,0 g (78,5 mM) des Racemats mit einer Lösung von 31,7 g (78,5 mM (-)-Di-O-toluoyl-L-weinsaure in 300 ml siedendem Ethanol. Die beim Abkühlen unter Rühren ausfallenden Kristalle (13,8 g) wurden unter Nachwaschen mit Ethanol abgesaugt und aus 200 ml Ethanol unter Zusatz von 200 ml Wasser umkristallisiert. Freisetzen der Base lieferte den (+)-Antipoden (5,5 g) mit [α]_{D} = + 97,0°
      (EtOH, c = 0,969).
      Aus der obigen Mutterlauge kristallisierten über Nacht 14,2 g eines Salzes, das aus 400 ml Ethanol (Abfiltrieren des unlöslichen Anteils in der Siedehitze) umkristallisiert wurde (Einengen auf 300 ml). Freisetzen der Base ergab 4,0 g des (-)-Antipoden, [α]_{D} = - 96,0°
      (EtOH, c = 0,940).
      Die exo-Phenyl-Konfigurationen wurden mittels Röntgenstrukturanalyse nachgewiesen.
   2. exo-6-Phenyl-3-azabicyclo[3.2.0]heptan
      50,0 g (28,9 mM) N-Cinnamyl-N-allylamin in 1600 ml Aceton wurden mit 300 ml 10 %iger Salzsaure versetzt und unter Stickstoff 48 h mit einer 150 Watt Quecksilber-Hochdrucklampe in einer Quarzapparatur bei Raumtemperatur bestrahlt. Anschließend engte man den Reaktionsansatz ein und verteilte den Ruckstand zwischen Methylenchlorid und Wasser. Man stellte mit waßriger Ammoniaklosung alkalisch und extrahierte die wäßrige Phase noch zweimal mit Methylenchlorid nach. Die vereinigten organischen Phasen wurden mit Natriumsulfat getrocknet und eingeengt. Ausbeute 49,0 g (98 %) viskoses Öl,
      Schmp. 177 bis 178°C (Maleinat).
   3. exo-6,7-Diphenyl-3-benzyl-3-azabicyclo[3.2.0]heptan
      70,0 g (206 mM) Bis-(N-Cinnamyl)-benzylamin in 2500 ml Aceton wurden mit 0,8 g Michlers Keton versetzt und unter Stickstoff 25 h mit einer 150 Watt Quecksilber-Hochdrucklampe in einer Duranglasapparatur bei Raumtemperatur bestrahlt. Anschließend engte man den Reaktionsansatz ein und verteilte den Rückstand zwischen Methylenchlorid und Wasser. Man stellte mit wäßriger Ammoniaklösung alkalisch und extrahierte die wäßrige Phase noch zweimal mit Methylenchlorid nach. Die vereinigten organischen Phasen wurden mit Natriumsulfat getrocknet und eingeengt. Die Reinigung des Rohproduktes (65,0 g) erfolgte durch Säulenchromatographie (Kieselgel, Laufmittel Toluol, Ethanol 98/2). Man erhielt 58,0 g (83 %) Produkt,
      Schmp.: 230-232°C (Hydrochlorid).
   4. exo-6,7-Diphenyl-3-azabicyclo[3.2.0]heptan
      Zu 12,0 g (35,4 mM) exo-6,7-Diphenyl-3-benzyl-3-azabicyclo[3.2.0]heptan in einer Mischung aus 300 ml n-Propanol und 16 ml Wasser wurden 16,0 g (254 mM) Ammoniumformiat sowie 2,0 g Palladium (10 %ig) auf Kohle gegeben und die Reaktionsmischung 4 h am Rückfluß gekocht (Entwicklung von Kohlendioxid). Nach dem Abkuhlen saugte man vom Katalysator ab, wusch mit Propanol und Methylenchlorid nach und engte das Filtrat ein. Man verteilte den Rückstand zwischen Methylenchlorid und Wasser, stellte mit wäßriger Ammoniaklösung alkalisch und extrahierte die wäßrige Phase noch zweimal mit Methylenchlorid nach. Die vereinigten organischen Phasen wurden mit Natriumsulfat getrocknet und eingeengt. Man erhielt 8,1 g (92 %) Produkt, Schmp. 140 bis 142°C (Maleinat).
   5. exo-6-Phenyl-3-benzyl-3-azabicyclo[3.2.0]heptan
      9,2 g (35,0 mM) N-Cinnamyl-N-allyl-benzylamin in 1100 ml Aceton wurden mit 100 mg Michlers Keton versetzt und unter Stickstoff 5 h mit einer 150 Watt Quecksilber-Hochdrucklampe in einer Duranglasapparatur bei Raumtemperatur bestrahlt. Anschließend engte man den Reaktionsansatz ein. Die Reinigung des Rohproduktes (9,4 g) erfolgte durch Säulenchromatographie (Kieselgel, Laufmittel Methylenchlorid/Methanol 98/2). Man erhielt 3,3 g (36 %) Produkt, Schmp.: 126-128°C (Maleinat).
   6. 2,2,2-Trifluoro-1-[exo-6-(3-pyridyl)-3-azabicyclo[3.2.0]hept-3-yl]-ethanon
      14,0 g (51,8 mM) N-Allyl,2,2,2-trifluoro-N-[3-(3-pyridyl)-allyl]-acetamid wurden in 140 ml Aceton gelöst, mit 30 ml 10 %iger wäßriger Salzsäure versetzt und unter Stickstoff 48 h mit einer 150 Watt Quecksilber-Hochdrucklampe in einer Duranglasapparatur bei Raumtemperatur bestrahlt. Danach wurde die Reaktionslösung eingeengt, in 150 ml Wasser aufgenommen und mit wäßriger Ammoniaklösung auf pH 8-9 eingestellt. Die wäßrige Phase wurde zweimal mit tert.-Butylmethylether extrahiert, die vereinigten organischen Phasen über Natriumsulfat getrocknet und eingeengt. Der verbleibende Rückstand wurde über Säulenchromatographie (Kieselgel, Methylenchlorid + 2 % Methanol) fraktioniert. Man erhielt 6,2 g (42 %) unverändertes N-Allyl-2,2,2-trifluoro-N-[3-(3-pyridyl)-allyl]-acetamid und 3,7 g (26 %) 2,2,2-Trifluoro-1-[exo-6-(3-pyridyl)-3-azabicyclo[3.2.0)hept-3-yl]-ethanon als dunkles Öl.
   7. exo-6-(3-Pyridyl)-3-azabicyclo[3.2.0]heptan
      Zur Lösung von 3,7 g (13,7 mM) 2,2,2-Trifluoro-1-[exo-6-(3-pyridyl)-3-azabicyclo[3.2.0]hept-3-yl]-ethanon in 50 ml Ethanol wurden 2,5 g Kaliumhydroxid-Plätzchen gegeben. Die Reaktionslösung wurde noch 2 h bei Raumtemperatur nachgerührt und anschließend auf 100 ml Eiswasser gegossen. Die wäßrige Phase wurde dreimal mit tert.-Butyl-methylether extrahiert, die vereinigten organischen Phasen wurden uber Natriumsulfat getrocknet und eingeengt. Ausbeute 2,3 (96 %) gelbes Öl,
      Schmp. 202-205°C (Hydrochlorid).
      Analog lassen sich folgende Substanzen herstellen:
   8. exo-6-(m-Fluor-phenyl)-3-azabicyclo[3.2.0]heptan
   9. exo-6-(o-Fluor-phenyl)-3-azabicyclo[3.2.0]heptan,
      Schmp. 118-120°C (Maleinat)
   10. exo-6-(p-Chlor-phenyl)-3-azabicyclo[3.2.0]heptan,
      Schmp. 152-154°C (Maleinat)
   11. exo-6-(m-Chlor-phenyl)-3-azabicyclo[3.2.0]heptan
      Schmp. 130-132°C (Maleinat)
   12. exo-6-(p-Methoxy-phenyl)-3-azabicyclo[3.2.0]heptan
   13. exo-6-(m-Methoxy-phenyl)-3-azabicyclo[3.2.0]heptan
   14. exo-6-(p-Nitro-phenyl)-3-azabicyclo[3.2.0]heptan
      Schmp. 158-160°C (Maleinat)
   15. exo-6-(m-Nitro-phenyl)-3-azabicyclo[3.2.0]heptan
   16. exo-6-(p-Trifluormethyl-phenyl)-3-azabicyclo-[3.2.0]heptan, Schmp. 155-156°C (Maleinat)
   17. exo-6-(m-Trifluormethyl-phenyl)-3-azabicyclo-[3.2.0]heptan
   18. exo-6-(3,4-Difluor-phenyl)-3-azabicyclo-[3.2.0]-heptan
   19. exo-6-(3,5-Dichlor-phenyl)-3-azabicyclo-[3.2.0]-heptan, Schmp. > 250°C (Hydrochlorid)
   20. exo-6-(3,4-Dimethoxy-phenyl))-3-azabicyclo-[3.2.0]-heptan
   21. exo-6-(m-Hydroxy-phenyl)-3-azabicyclo-[3.2.0]heptan
   22. exo-6-(p-Hydroxy-phenyl)-3-azabicyclo-[3.2.0]heptan
   23. exo-6-(3,4-Dihydroxy-phenyl)-3-azabicyclo-[3.2.0]-heptan
   24. exo-6-(p-Methyl-phenyl)-3-azabicyclo-[3.2.0]heptan
   25. exo-6-(m-Methyl-phenyl)-3-azabicyclo-[3.2.0]heptan
   26. exo-6-(p-t-Butyl-phenyl)-3-azabicyclo-[3.2.0]heptan,
      Schmp. > 255°C (Hydrochlorid)
   27. exo-6-(m-Amino-phenyl)-3-azabicyclo-[3.2.0]heptan
   28. exo-6-(p-Amino-phenyl)-3-azabicyclo-[3.2.0]heptan
   29. exo-6-(p-Cyano-phenyl)-3-azabicyclo-[3.2.0]heptan,
      Schmp. 168-170°C (Maleinat)
   30. exo-6-Thien-2-yl-3-azabicyclo-[3.2.0]heptan,
      Schmp. 180-182°C (Hydrochlorid)
   31. exo-6-Thien-3-yl-3-azabicyclo-[3.2.0]heptan,
      Schmp. 143-145°C (Hydrochlorid)
   32. exo-6-(5-Chlor-thien-2-yl)-3-azabicyclo-[3.2.0]-heptan, Schmp. 156-157°C (Maleinat)
B) Herstellung der Endprodukte

### Beispiel 1

### N-(2-[exo-6-p-Fluor-phenyl-3-azabicyclo[3.2.0]heptan-3-yl]ethyl)-benzosulfonamid Hydrochlorid

3,0 g (15,7 mM) exo-6-p-Fluor-phenyl-3-azabicyclo[3.2.0]heptan in 60 ml Xylol wurden mit 3,5 g (15,7 mM)N-(2-Chlorethyl)-benzosulfonamid sowie mit 2,2 g (15,7 mM) fein pulverisiertem Kaliumcarbonat nebst 0,5 g Kaliumiodid versetzt und unter gutem Rühren 4 h unter Rückfluß gekocht.

Nach dem Abkühlen engte man am Rotationsverdampfer ein und verteilte den Rückstand zwischen Methylenchlorid und Wasser (pH = 10).

Die wäßrige Phase wurde zweimal mit Methylenchlorid nachextrahiert und darauf die organische Phase nach einmaligem Nachwaschen mit Wasser und Trocknen mit Natriumsulfat eingeengt. Das Rohprodukt (7,6 g) reinigte man durch Säulenchromatographie (Kieselgel, Laufmittel Methylenchlorid/Methanol 98/2). Die freie Base nahm man in 30 ml Essigester auf, filtrierte die unlöslichen Flocken ab und versetzte die Etherlosung mit überschüssiger etherischer Salzsaure. Nach 1 h Rühren gab man 150 ml Ether hinzu und ließ über Nacht stehen. Anschließend saugte man die Festkörper in der Kälte ab und wusch das Hydrochlorid mit reichlich Ether nach. Man isolierte 4,1 g (64 %) Produkt, Schmp. 133 bis 135°C.

Analog lassen sich herstellen:
2. N-(2-[exo-6-Phenyl-3-azabicyclo[3.2.0]heptan-3-yl]-ethyl)-benzosulfonamid, Schmp. 116-118°C (Hydrochlorid),
3. N-(2-[exo-6-p-Fluor-phenyl-3-azabicyclo-[3.2.0]-heptan-3-yl]-ethyl)-N-methyl-benzosulfonamid, Schmp. 63-65°C (Hydrochlorid),
4. N-(2-[exo-6-(5-Chlor-thien-2-yl)-3-azabicyclo[3.2.0]-heptan-3-yl]-ethyl)-4-fluor-benzosulfonamid,
5. 3-[exo-6-p-Fluor-phenyl-3-azabicyclo[3.2.0]heptan-3-yl]-1-phenoxy-propan, Schmp. 128-130°C (Hydrochlorid),
6. 2-[exo-6-p-Fluor-phenyl-3-azabicyclo[3.2.0]heptan-3-yl]-1-p-fluor-phenoxy-ethan, Schmp. 177-178°C (Hydrochlorid),
7. 3-[exo-6-p-Fluor-phenyl-3-azabicyclo[3.2.0]heptan-3-yl]-1-(1-naphthyloxy)-propan,
8. 3-[exo-6-p-Fluor-phenyl-3-azabicyclo[3.2.0]heptan-3-yl]-1-p-t-butyl-phenoxy-propan,
9. 3-[exo-6-p-Fluor-phenyl-3-azabicyclo-[3.2.0]heptan-3-yl]-1-p-fluor-phenoxy-propan, Schmp. 144-146°C (Tosylat).

### Beispiel 10

### N-(2-[exo-6-p-Fluor-phenyl-3-azabicyclo-[3.2.0]heptan-3-yl]-ethyl)-thiobenzamid Tosylat

2,4 g (7,1 mM) N-(2-[exo-6-p-Fluor-phenyl-3-azabicyclo[3.2.0]heptan-3-yl]-ethyl-benzamid (DE 42 19 973) in 50 ml Toluol wurden mit 1,5 g (3,6 mM) Lawesson-Ragens versetzt und unter gutem Rühren 3 h unter Rückfluß gekocht. Nach dem Abkühlen engte man am Rotationsverdampfer ein, verteilte den Rückstand zwischen Methylenchlorid und Wasser und stellte mit 10 %iger Natronlauge alkalisch. Die wäßrige Phase wurde zweimal mit Methylenchlorid nachextrahiert und darauf die organische Phase nach dem Trocknen mit Natriumsulfat eingeengt. Das Rohprodukt (3,5 g) reinigte man durch Säulenchromatographie (Kieselgel, Laufmittel Methylenchlorid/Methanol 99/1). Man löste die gereinigte freie Base in 150 ml Ether und tropfte unter Eiskühlung und Rühren eine Losung von 1,0 g p-Toluolsulfonsäure in Essigester langsam zu. Das ausgefallene Salz wurde unter Stickstoff abgesaugt, mit Ether nachgewaschen und unter Stickstoff getrocknet. Man isolierte 2,7 g (72 %) Produkt als Tosylat, Schmp. 119-122°C.

Analog lassen sich herstellen:
11. N-(2-[exo-6-p-Fluor-phenyl-3-azabicyclo[3.2.0]heptan-3-yl]-ethyl)-4-fluor-thiobenzamid
12. N-(2-[exo-6-p-Fluor-phenyl-3-azabicyclo[3.2.0]heptan-3-yl]-ethyl)-4-chlor-thiobenzamid

### Beispiel 13

### O-(2-[exo-6-p-Fluor-phenyl-3-azabicyclo[3.2.0]heptan-3-yl]-ethyl)-benzoat Maleinat

a) Zu einer Losung von 11,5 g (142 mM) Chlorethanol in 200 ml THF tropfte man unter gutem Ruhren bei Raumtemperatur 20,0 g (142 mM) Benzoylchlorid und anschließend noch 14,4 g (142 mM) Triethylamin hinzu (exotherme Reaktion). Nach 1 h Nachrühren engte man am Rotationsverdampfer ein, verteilte den Rückstand zwischen Methylenchlorid und Wasser und sauerte mit 10 %iger Salzsäure an. Die wäßrige Phase wurde zweimal mit Methylenchlorid nachextrahiert und darauf die organische Phase nach dem Trocknen mit Natriumsulfat eingeengt. Man isolierte 26,0 g (99 %) 2-Chlor-ethyl-benzoat
b) 3,0 g (15,7 mM) exo-6-(p-Fluor-phenyl)-3-azabicyclo[3.2.0]heptan in 50 ml Toluol wurden mit 6,0 g (32 mM) 2-Chlor-ethyl-benzoat sowie mit 2,2 g (16 mM) fein pulverisiertem Kaliumcarbonat nebst 0,4 g Kaliumjodid versetzt und unter gutem Rühren 15 h unter Rückfluß gekocht. Nach dem Abkühlen engte man am Rotationsverdampfer ein und verteilte den Rückstand zwischen Methylenchlorid und Wasser. Die wäßrige Phase wurde nach Einstellen auf pH = 10 zweimal mit Methylenchlorid nachextrahiert und darauf die organische Phase nach dem Trocknen mit Natriumsulfat eingeengt. Das Rohprodukt (8,9 g) reinigte man durch Saulenchromatographie (Kieselgel, Laufmittel Methylenchlorid/Methanol 99/1). Man loste die gereinigte freie Base (2,8 g) in 150 ml Ether und tropfte unter Eiskühlung und Rühren eine Lösung von 1,0 g Maleinsäure in 10 ml Aceton langsam zu. Das ausgefallene Salz wurde unter Stickstoff abgesaugt, mit Ether nachgewaschen und unter Stickstoff getrocknet. Man isolierte 3,9 g (53 %) Produkt als Maleinat, Schmp. 139-141°C.

Analog läßt sich herstellen:
14. O-(2-[exo-6-p-Fluor-phenyl-3-azabicyclo[3.2.0]heptan-3-yl]-ethyl-4-fluor-benzoat.

### Beispiel 15

### N-(2-[exo-6-p-Fluor-phenyl-3-azabicyclo[3.2.0]heptan-3-yl]ethyl)-1H-benzo[cd]indol-2-on

2,5 g (13,1 mM) exo-6-p-Fluor-phenyl-3-azabicyclo[3.2.0]heptan in 50 ml Xylol wurden mit 3,0 g (13,1 mM) 1-(2-Chlorethyl)-1H-benzo[cd]indol-2-on sowie mit 1,9 g (13,1 mM) fein pulverisiertem Kaliumcarbonat und 0,5 g Kaliumjodid versetzt und unter gutem Ruhren 3 h unter Ruckfluß gekocht. Nach dem Abkühlen engte man am Rotationsverdampfer ein und verteilte den Rückstand zwischen Methylenchlorid und Wasser (pH = 10). Die wäßrige Phase wurde zweimal mit Methylenchlorid nachextrahiert und darauf die organische Phase nach dem Trocknen mit Natriumsulfat eingeengt. Das Rohprodukt (6,0 g) reinigte man durch Säulenchromatographie (Kieselgel, Laufmittel Methylenchlorid/Methanol 98/2).

Man löste die freie Base (3,4 g) in wenig Essigsaureethylester und versetzte unter Eiskühlung mit überschüssiger etherischer Salzsäure. Durch Zugabe von Diethylether wurde die Fällung vervollständigt und danach 10 min bei 0°C nachgerührt. Das ausgefallene Hydrochlorid wurde unter Stickstoff abgesaugt, mit Diethylether nachgewaschen und danach bei 40°C im Vakuumtrockenschrank getrocknet. Man erhielt so 2,7 g (49 %) helles Pulver, Schmp. > 250°C (Hydrochlorid).

Analog lassen sich herstellen:
16. N-(2-[exo-6-p-Chor-phenyl-3-azabicyclo[3.2.0]heptan-3-yl]-ethyl)-1H-benzo[cd]indol-2-on, Schmp. 233-235°C (Hydrochlorid),
17. N-(2-[exo-6-m-Fluor-phenyl-3-azabicyclo[3.2.0]heptan-3-yl]-ethyl)-1H-benzo[cd]indol-2-on,
18. N-(2-[exo-6-m-Chlor-phenyl-3-azabicyclo[3.2.0]heptan-3-yl]-ethyl)-1H-benzo[cd]indol-2-on,
19. 3,3-Dimethyl-1-(2-[exo-6-p-fluor-phenyl-3-azabicyclo[3.2.0]heptan-3-yl]-ethyl)-indolinon-2, Schmp. 198-200°C (Hydrochlorid),
20. 3,3-Dimethyl-1-(2-[exo-6-p-chlor-phenyl-3-azabicyclo[3.2.0]heptan-3-yl]-ethyl)-indolinon-2, Zersp. 102°C (Maleinat),
21. 3,3-Dimethyl-1-(2-[exo-6-m-chlor-phenyl-3-azabicyclo[3.2.0]heptan-3-yl]-ethyl)-indolinon-2,
22. 1-(2-[exo-6-p-Chlor-phenyl-3-azabicyclo[3.2.0]heptan-3-yl]-ethyl)-3,3,5-trimethyl-indolinon-2, Schmp. 227-229°C (Hydrochlorid),
23. 2-Amino-N-(2-[exo-6-p-fluor-phenyl-3-azabicyclo[3.2.0]heptan-3-yl]-ethyl)-benzamid, Schmp. 138-139°C,
24. 2-Amino-N-(2-[exo-6-p-chlor-phenyl-3-azabicyclo[3.2.0]heptan-3-yl]-ethyl)-benzamid, Schmp. 127-128°C,
25. N-(2-[exo-6-p-Fluor-phenyl-3-azabicyclo[3.2.0]heptan-3-yl]-ethyl)-2-methylamino-benzamid, Schmp. 105-110°C (Dihydrochlorid),
26. N-(2-[exo-6-p-Chlor-phenyl-3-azabicyclo[3.2.0]heptan-3-yl]-ethyl)-2-methylamino-benzamid, Zersp. 107°C (Dihydrochlorid),
27. 3-Amino-N-(2-[exo-6-p-chlor-phenyl-3-azabicyclo[3.2.0]heptan-3-yl]-ethyl)-benzamid,
28. 4-Amino-N-(2-[exo-6-p-chlor-phenyl-3-azabicyclo[3.2.0]heptan-3-yl]-ethyl)-benzamid,
29. N-(2-[exo-6-p-Fluor-phenyl-3-azabicyclo[3.2.0]heptan-3-yl]-ethyl)-thiophen-2-carbonsäureamid, Schmp. 185-186°C (Hydrochlorid),
30. 5-Chlor-N-(2-[exo-6-p-fluor-phenyl-3-azabicyclo[3.2.0]heptan-3-yl]-ethyl)-thiophen-2-carbonsäureamid, Schmp. 129-131°C,
31. 5-Chlor-N-(2-[exo-6-p-chlor-phenyl-3-azabicyclo[3.2.0]heptan-3-yl]-ethyl)-thiophen-2-carbonsäureamid, Schmp. 136-138°C,
32. 5-Chlor-N-(2-[exo-6-(5-chlor-2-thienyl)-3-azabicyclo[3.2.0]heptan-3-yl]-ethyl)-thiophen-2-carbonsäureamid,
33. N-(2-[exo-6-p-Fluor-phenyl-3-azabicyclo[3.2.0]heptan-3-yl]-ethyl)-benzo[b]furan-2-carbonsäureamid,
   Schmp. 250-251°C (Hydrochlorid),
34. 3-Chlor-N-(2-[exo-6-p-fluor-phenyl-3-azabicyclo[3.2.0]heptan-3-yl]-ethyl)-benzo[b]thiophen-2-carbonsäureamid, Schmp. 104-106°C,
35. 3-Chlor-N-(2-[exo-6-p-chlor-phenyl-3-azabicyclo[3.2.0]heptan-3-yl]-ethyl)-benzo[b]thiophen-2-carbonsäureamid,
36. 3-Chlor-N-(2-[exo-6-p-nitro-phenyl-3-azabicyclo[3.2.0]heptan-3-yl]-ethyl)-benzo[b]thiophen-2-carbonsäureamid,
37. N-(2-[exo-6-p-Fluor-phenyl-3-azabicyclo[3.2.0]heptan-3-yl]-ethyl)-inden-3-carbonsäureamid, Schmp. 107-109°C,
38. N-(2-[exo-6-p-Chlor-phenyl-3-azabicyclo[3.2.0]heptan-3-yl]-ethyl)-inden-3-carbonsäureamid,
39. N-(2-[exo-6-p-Fluor-phenyl-3-azabicyclo[3.2.0]heptan-3-yl]-ethyl)-cyclopropan-carbonsäureamid, Schmp. 104-105°C,
40. N-(2-[exo-6-p-Fluor-phenyl-3-azabicyclo[3.2.0]heptan-3-yl]-ethyl)-cyclopentan-carbonsäureamid, Schmp. 78-82°C,
41. N-(2-[exo-6-p-Chlor-phenyl-3-azabicyclo[3.2.0]heptan-3-yl]-ethyl)-cyclopentan-carbonsäureamid,
42. N-(2-[exo-6-p-Fluor-phenyl-3-azabicyclo[3.2.0]heptan-3-yl]-ethyl)-cyclohexan-carbonsäureamid, Schmp. 111-113°C,
43. N-(2-[exo-6-p-Chlor-phenyl-3-azabicyclo[3.2.0]heptan-3-yl]-ethyl)-cyclohexan-carbonsäureamid, Schmp. 106-107°C,
44. N-(2-[exo-6-p-Fluor-phenyl-3-azabicyclo[3.2.0]heptan-3-yl]-ethyl)-naphthalin-1-carbonsäureamid, Schmp. 202-204°C (Hydrochlorid),
45. N-(2-[exo-6-p-Fluor-phenyl-3-azabicyclo[3.2.0]heptan-3-yl]-ethyl)-indol-2-carbonsäureamid,
46. N-(2-[exo-6-p-Chlor-phenyl-3-azabicyclo[3.2.0]heptan-3-yl]-ethyl)-indol-3-carbonsäureamid,

### Beispiel 47

### exo-6-p-Fluorphenyl-3-[2-(1-naphthyl)-ethyl]-3-azabicyclo[3.2.0]heptan

2,5 g (13,1 mM) exo-6-p-Fluorphenyl-3-azabicyclo[3.2.0]heptan in 50 ml Xylol wurden mit 3,2 g (13,6 mM) 1-(2-Bromethyl)-naphthalin sowie mit 1,9 g (13,1 mM) fein pulverisiertem Kaliumcarbonat und 0,5 g Kaliumjodid versetzt und unter gutem Rühren 2 h unter Rückfluß gekocht. Nach dem Abkühlen engte man am Rotationsverdampfer ein und verteilte den Rückstand zwischen Methylenchlorid und Wasser (pH = 10). Die wäßrige Phase wurde zweimal und Methylenchlorid nachextrahiert und darauf die organische Phase nach dem Trocknen mit Natriumsulfat eingeengt. Das Rohprodukt (6,7 g) reinigte man durch Säulenchromatographie (Kieselgel, Laufmittel Methylenchlorid/Methanol 97,5/2,5).

Man löste die freie Base (3,3 g) in Diethylether und wenig Essigsäureethylester und versetzte unter Eiskühlung mit überschüssiger etherischer Salzsäure. Das ausgefallene Hydrochlorid wurde unter Stickstoff abgesaugt, mit Diethylether nachgewaschen und danach bei 40°C im Vakuumtrockenschrank getrocknet. Man erhielt so 1,2 g (24 %) weißes, feines Pulver, Schmp. 212-214°C (Hydrochlorid).

Analog lassen sich herstellen:
48. exo-6-p-Chlor-phenyl-3-[2-(1-naphthyl)-ethyl]-3-azabicyclo[3.2.0]heptan, Schmp. 215-216°C (Hydrochlorid),
49. exo-6-m-Chlor-phenyl-3-[2-(1-naphthyl)-ethyl]-3-azabicyclo[3.2.0]heptan, Schmp. 185-187°C (Hydrochlorid),
50. exo-6-(5-Chlor-2-thienyl)-3-[2-(1-naphthyl)-ethyl]-3-azabicyclo[3.2.0]heptan, Schmp. 209-210°C (Hydrochlorid),
51. exo-6-p-Fluor-phenyl-3-[2-(2-naphthyl)-ethyl]-3-azabicyclo[3.2.0]heptan, Schmp. 163-164°C (Hydrochlorid),
52. exo-6-p-Fluor-phenyl-3-[1-naphthyl-methyl]-3-azabicyclo[3.2.0]heptan, Schmp. 114-116°C (Maleinat),
53. exo-6-p-Fluor-phenyl-3-[2-naphthyl-methyl]-3-azabicyclo[3.2.0]heptan, Schmp. 153-155°C (Hydrochlorid),
54. 4-(6-p-Fluor-phenyl-3-azabicyclo[3.2.0]heptan-3-yl)-1-(thiophen-2-yl)-butan-1-on, Schmp. 197-199°C (Hydrochlorid),
55. 4-(6-p-Chlor-phenyl-3-azabicyclo[3.2.0]heptan-3-yl)-1-(thiophen-2-yl)-butan-1-on,
   Schmp. 176-177°C (Hydrochlorid).

### Beispiel 56

### N-(2-[exo-6-p-Fluor-phenyl-3-azabicyclo-[3.2.0]heptan-3-yl]-ethyl)-4-phenyl-pyrrolidinon-2 Tartrat

a) 25,0 g (131 mM) exo-6-(p-Fluor-phenyl)-3-azabicyclo[3.2.0]heptan in 350 ml THF wurden mit 74,0 g (523 mM) 1-Brom-2-chlor-ethan sowie mit 18,0 g (131 mM) fein pulverisiertem Kaliumcarbonat versetzt und unter gutem Rühren 15 h unter Rückfluß gekocht. Nach dem Abkühlen engte man am Rotationsverdampfer ein und verteilte den Rückstand zwischen Methyl-t-butyl-ether und Wasser.
   Die wäßrige Phase wurde nach Einstellen auf pH = 10 zweimal mit Methyl-t-butyl-ether nachextrahiert und darauf die organische Phase nach dem Trocknen mit Natriumsulfat eingeengt. Das Rohprodukt (34,8 g) reinigte man durch Säulenchromatographie (Kieselgel, Laufmittel Methylenchlorid). Man isolierte 21,9 g (66 %) 3-(β-Chlorethyl)-exo-6-(p-fluor-phenyl)-3-azabicyclo-[3.2.0]heptan als hellgelbes Öl.
b) Zu 1,56 g (9,9 mM) 4-Phenyl-pyrrolidinon-2 in 30 ml DMF wurde unter Stickstoff bei Raumtemperatur 0,30 g (9,9 mM) 80 %iges Natriumhydrid eingetragen und die Mischung unter gutem Rühren 1 h auf 120°C geheizt. Nach dem Abkühlen gab man 2,5 g (9,9 mM) 3-(β-Chlor-ethyl)-exo-6-(p-fluor-phenyl)-3-azabicyclo-[3.2.0]heptan hinzu und ließ nochmals 2 h bei 140°C Badtemperatur nachrühren. Nach dem Abkühlen engte man am Rotationsverdampfer ein und verteilte den Rückstand zwischen Methyl-t-butyl-ether und Wasser. Die wäßrige Phase wurde nach Einstellen auf pH = 10 zweimal mit Methyl-t-butyl-ether nachextrahiert und darauf die organische Phase nach dem Trocknen mit Natriumsulfat eingeengt. Das Rohprodukt (3,5 g) reinigte man durch Säulenchromatographie (Kieselgel, Laufmittel Methylenchlorid/Methanol 97/3). Man löste die gereinigte freie Base (2,5 g) in 150 ml Ether und tropfte unter Eiskühlung und Rühren eine Lösung von 1,0 g Weinsäure in 10 ml Ethanol langsam zu. Das ausgefallene Salz wurde unter Stickstoff abgesaugt, mit Ether nachgewaschen und unter Stickstoff getrocknet. Man isolierte 3,2 g (61 %) Produkt als Tartrat, Schmp. 74-77°C.

### Beispiel 57

### N-(2-[exo-6-p-Fluor-phenyl-3-azabicyclo-[3.2.0]heptan-3-yl]-ethyl)-benzoxazolinon-2

a) 10 g (74 mM) Benzoxazolinon-2 in 150 ml 1,2-Dichlorethan wurden mit 7,1 g (111 mM) Kaliumhydroxidpulver (88 %) sowie 0,5 g Benzyl-triethyl-ammoniumchlorid (TEBAC) versetzt und unter gutem Rühren 4 h unter Rückfluß gekocht. Nach dem Abkühlen engte man am Rotationsverdampfer ein und verteilte den Rückstand zwischen Methylenchlorid und Wasser.
   Die wäßrige Phase wurde zweimal mit Methylenchlorid nachextrahiert und darauf die organische Phase nach dem Trocknen mit Natriumsulfat eingeengt. Das Rohprodukt (11,7 g) reinigte man durch Säulenchromatogrpahie (Kieselgel, Laufmittel Methylenchlorid). Man isolierte 8,2 g (56 %) N-(2-Chlor)-ethyl-benzoxazolinon-2.
b) 2,5 g (13,1 mM) exo-6-(p-Fluor-phenyl)-3-azabicyclo[3.2.0]heptan in 40 ml Xylol wurden mit 3,0 g (15,2 mM) N-(2-Chlor)-ethyl-benzoxazolinon-2 sowie mit 1,8 g (13,1 mM) fein pulverisiertem Kaliumcarbonat nebst 0,3 g Kaliumjodid versetzt und unter gutem Rühren 7 h unter Rückfluß gekocht. Nach dem Abkühlen engte man am Rotationsverdampfer ein und verteilte den Rückstand zwischen Methylenchlorid und Wasser.
   Die wäßrige Phase wurde nach Einstellen auf pH = 9 zweimal mit Methylenchlorid nachextrahiert und darauf die organische Phase nach dem Trocknen mit Natriumsulfat eingeengt. Das Rohprodukt (6,5 g) reinigte man durch Säulenchromatographie (Kieselgel, Laufmittel Methylenchlorid/Methanol 98/2). Man isolierte 3,5 g (76 %) Produkt, Schmp. 138-140°C (Fumarat).

### Beispiel 58

### N-(2-[exo-6-p-Fluor-phenyl-3-azabicyclo-[3.2.0]heptan-3-yl]-ethyl)-isoindolinon

a) 13,3 g (100 mM) Phthalimidin in 200 ml 1,2-Dichlorethan wurden mit 9,6 g (150 mM) Kaliumhydroxidpulver (88 %) sowie 0,5 g Benzyl-triethyl-ammoniumchlorid (TEBAC) versetzt und unter gutem Rühren 5 h unter Rückfluß gekocht. Nach dem Abkühlen engte man am Rotationsverdampfer ein und verteilte den Rückstand zwischen Methylenchlorid und Wasser.
   Die wäßrige Phase wurde nach Einstellen auf pH = 6 zweimal mit Methylenchlorid nachextrahiert und darauf die organische Phase nach dem Trocknen mit Natriumsulfat eingeengt. Das Rohprodukt (15,0 g) reinigte man durch Säulenchromatographie (Kieselgel, Laufmittel Methylenchlorid/Methanol 97/3). Man isolierte 9,8 g (50 %) N-(2-Chlor)-ethyl-isoindolinon.
b) 2,5 g (13,1 mM) exo-6-(p-Fluor-phenyl)-3-azabicyclo[3.2.0]heptan in 50 ml Xylol wurden mit 2,75 g (14,0 mM) N-(2-Chlor)-ethyl-isoindolinon sowie mit 2,0 g (14,0 mM) fein pulverisiertem Kaliumcarbonat nebst 0,5 g Kaliumjodid versetzt und unter gutem Rühren 8 h unter Rückfluß gekocht. Nach dem Abkühlen engte man am Rotationsverdampfer ein und verteilte den Rückstand zwischen Methylenchlorid und Wasser.
   Die wäßrige Phase wurde nach Einstellen auf pH = 10 zweimal mit Methylenchlorid nachextrahiert und darauf die organische Phase nach dem Trocknen mit Natriumsulfat eingeengt. Das Rohprodukt (5,6 g) reinigte man durch Säulenchromatographie (Kieselgel, Laufmittel Methylenchlorid/Methanol 96/4). Man isolierte 3,5 g (76 %) Produkt, Schmp. 223-225°C (Hydrochlorid).

Analog Beispiel 58 wurde hergestellt:
59. 1-(2-[exo-6-p-Fluor-phenyl-3-azabicyclo[3.2.0]heptan-3-yl]-ethyl)-indazol, Schmp. 164-166°C (Hyrochlorid),
60. 1-(2-[exo-6-p-Fluor-phenyl-3-azabicyclo[3.2.0]heptan-3-yl]-ethyl)-1,8-naphthalinsultam, Schmp. 206-208°C (Hydrochlorid),
61. 4-(2-[exo-6-p-Fluor-phenyl-3-azabicyclo[3.2.0]heptan-3-yl]-ethyl)-1,4-benzoxazin-3-on, Schmp. 166-168°C (Tosylat),
62. 1-(2-[exo-6-p-Fluor-phenyl-3-azabicyclo[3.2.0]heptan-3-yl]-ethyl)-chinoxalin-2(1H)-on, Schmp. 54-56°C (Tartrat)

### Beispiel 63

### 1-(2-[exo-6-p-Fluor-phenyl-3-azabicyclo[3.2.0]heptan-3-yl]-ethyl)-indolinon-2

a) 13,3 g (100 mM) Oxindol in 150 ml 1,2-Dichlorethan wurden mit 11,0 g (173 mM) Kaliumhydroxidpulver (88 %) sowie 0,5 g Benzyl-triethyl-ammoniumchlorid (TEBAC) versetzt und unter gutem Rühren 6 h unter Rückfluß gekocht. Nach dem Abkühlen engte man am Rotationsverdampfer ein und verteilte den Rückstand zwischen Methylenchlorid und Wasser.
   Die wäßrige Phase wurde zweimal mit Methylenchlorid nachextrahiert und darauf die organische Phase nach dem Trocknen mit Natriumsulfat eingeengt. Das Rohprodukt (15,5) g reinigte man durch Säulenchromatographie (Kieselgel, Laufmittel Methylenchlorid/Methanol 99/1). Man isolierte 7,1 g eines Gemisches von N-(2-Chlor)-ethyl-oxindol und des entsprechenden Spirocyclopropyl-Derivates.
b) 3,4 g (17,8 mM) exo-6-(p-Fluor-phenyl)-3-azabicyclo[3.2.0]heptan in 50 ml Xylol wurden mit 5,2 g des obigen Produktgemisches sowie mit 3,3 g (24,0 mM) fein pulverisiertem Kaliumcarbonat nebst 0,5 g Kaliumjodid versetzt und unter gutem Rühren 9 h unter Rückfluß gekocht. Nach dem Abkühlen engte man am Rotationsverdampfer ein und verteilte den Rückstand zwischen Methylenchlorid und Wasser.
   Die wäßrige Phase wurde nach Einstellen auf pH = 10 zweimal mit Methylenchlorid nachextrahiert und darauf die organische Phase nach dem Trocknen mit Natriumsulfat eingeengt. Das Rohprodukt (8,4 g) reinigte man durch Säulenchromatographie (Kieselgel, Laufmittel Methylenchlorid/Methanol 99/1). Man isolierte 3,5 g eines Produktgemisches (1:1), das nochmals zur Feinauftrennung einer Säulenchromatographie (Kieselgel, Laufmittel n-Hexan/Essigester 1/1) unterworfen wurde.
   Man isolierte als polare Substanz 1,9 g (31 %) 1-(2-[exo-6-p-Fluor-phenyl-3-azabicyclo[3.2.0]heptan-3-yl]-ethyl)-indolinon-2, Schm. 91-93°C (Tartrat).

### Beispiel 64

### 1-(2-[exo-6-p-Fluor-phenyl-3-azabicyclo[3.2.0]heptan-3-yl]-ethyl)-spiro[cyclopropan-1',3-indolinon-2]

Als unpolare Komponente des Produktgemisches aus Beispiel 65b isolierte man 1,4 g (21 %) 1-(2-[exo-6-p-Fluor-phenyl-3-azabicyclo[3.2.0]heptan-3-yl]-ethyl)-spiro[cyclopropan-1',3-indolinon-2], das sich nach Überführung ins Tartrat-Salz ab 129°C zersetzte.

## Patentansprüche

1. N-substituierte 3-Azabicyclo[3.2.0]-heptan-Derivate der Formel I 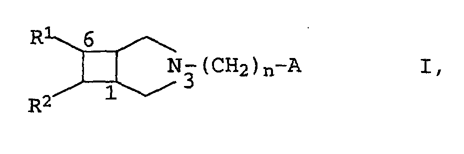 worin
R¹ eine gegebenenfalls durch Halogenatome, C₁-C₄-Alkyl, Trifluormethyl-, Hydroxy-, C₁-C₄-Alkoxy-, Amino-, Mono- methylamino-, Dimethylamino-, Cyano- oder Nitrogruppen mono- oder disubstituierte Phenyl- oder Thienylgruppe bedeutet,
R² ein Wasserstoffatom oder eine gegebenenfalls durch Halogen, Methoxy, Hydroxy oder Amino substituierte Phenylgruppe ist,
n die Zahl 1, 2, 3 oder 4 bedeutet,
A ein Wasserstoffatom oder einer der Reste 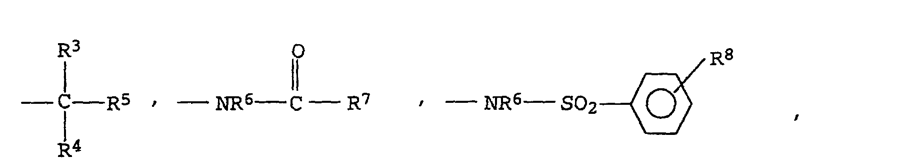 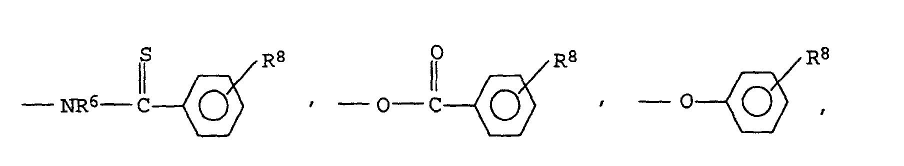 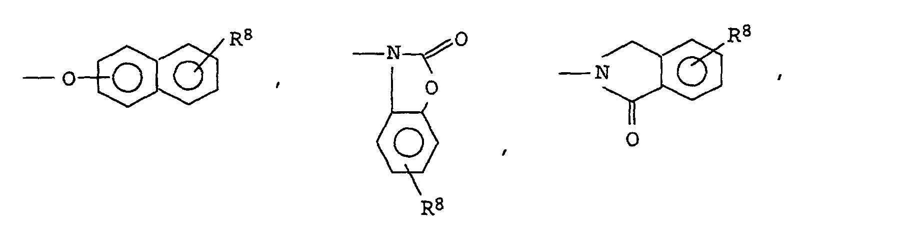 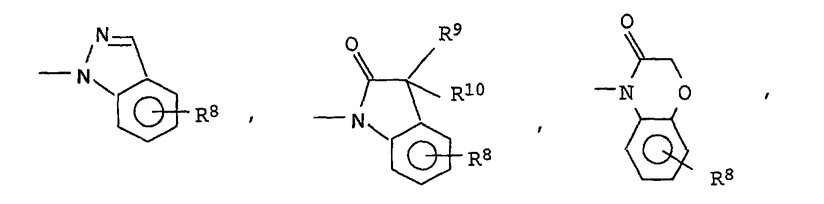 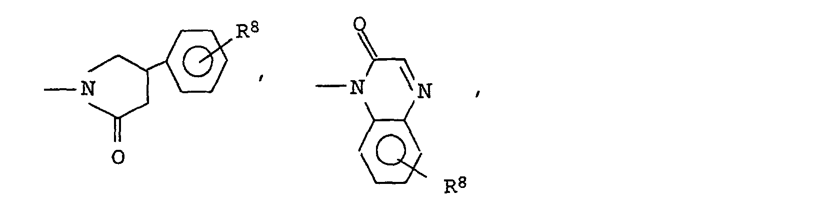 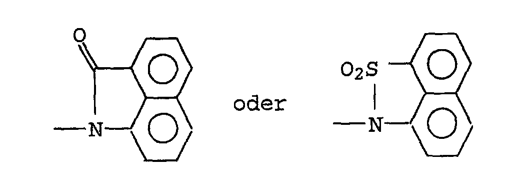 ist,
R³ ein Wasserstoffatom oder einen Hydroxyrest darstellt,
R⁴ ein Wasserstoffatom bedeutet oder
R³ und R⁴ zusammen ein Sauerstoffatom darstellen,
R⁵ eine gegebenenfalls durch Fluor oder Chlor substituierte Thienyl- oder Naphthylgruppe bedeutet,
R⁶ ein Wasserstoffatom oder eine Methylgruppe darstellt,
R⁷ eine durch durch Amino, C₁₋₄-Alkylamino oder Di-C₁₋₄-alkylamino monosubstituierte Phenylgruppe oder eine gegebenenfalls durch Fluor, Chlor oder Nitro substituierte Thienyl-, Naphthyl-, Benzofuryl-, Benzothienyl-, Indolyl-, N-Methylindolyl- oder Indenylgruppe oder eine C₃- bis C₆-Cycloalkylgruppe bedeutet,
R⁸ Wasserstoff, Fluor, Chlor, C₁₋₄-Alkyl, Methoxy oder Amino bedeutet,
R⁹ Wasserstoff oder eine Methylgruppe darstellt und
R¹⁰ Wasserstoff oder eine Methylgruppe darstellt oder
R⁹ und R¹⁰ zusammen mit dem Ring-C-Atom einen Spirocyclopropanring darstellen,
und deren Salze mit physiologisch verträglichen Säuren.

2. N-substituierte 3-Azabicyclo[3.2.0)heptan-Derivate der Formel I gemäß Anspruch 1 zur Verwendung bei der Bekämpfung von Krankheiten.

3. Verwendung der Verbindungen der Formel I gemäß Anspruch 1 zur Herstellung von Arzneimitteln.

4. Verwendung der Verbindungen der Formel I gemäß Anspruch 1 zur Herstellung von Arzneimitteln zur Behandlung von Erkrankungen des Zentralen Nervensystems.

## Claims

1. N-Substituted 3-azabicyclo[3.2.0]-heptane derivatives of the formula I 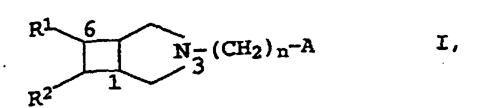 wherein
R¹ denotes a phenyl or thienyl group which is optionally mono- or disubstituted by halogen atoms, C₁-C₄-alkyl or trifluoromethyl, hydroxyl, C₁-C₄-alkoxy, amino, monomethylamino, dimethylamino, cyano or nitro groups,
R² is a hydrogen atom or a phenyl group which is optionally substituted by halogen, methoxy, hydroxyl or amino,
n denotes the number 1, 2, 3 or 4,
A is a hydrogen atom or one of the radicals 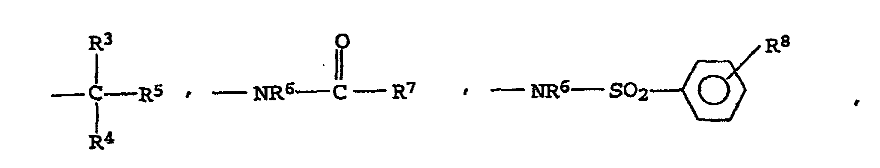 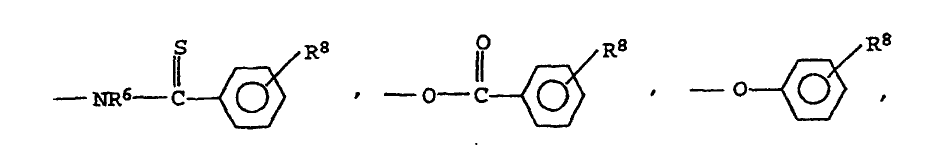 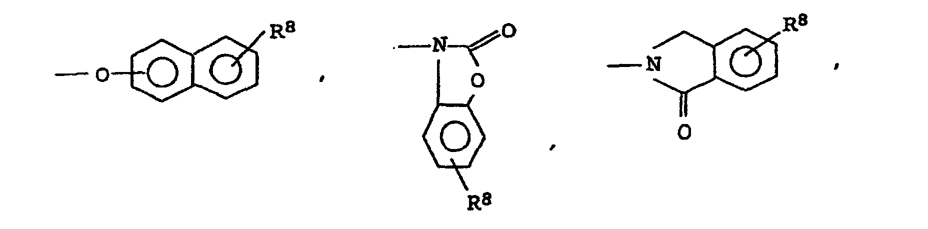 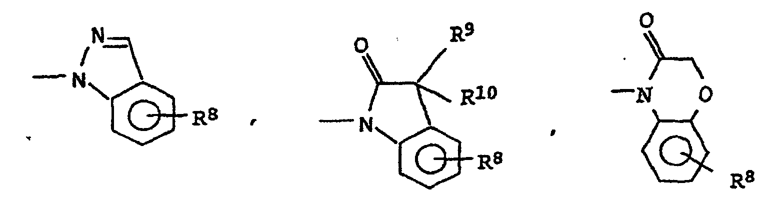 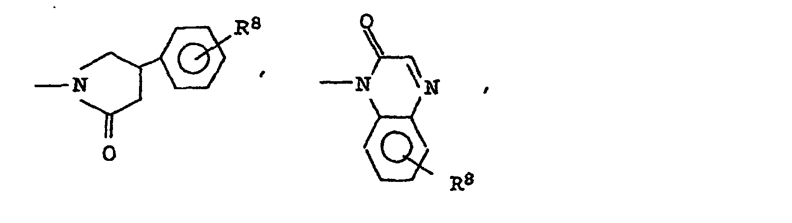 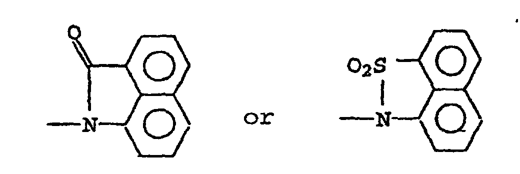
R³ represents a hydrogen atom or a hydroxyl radical,
R⁴ denotes a hydrogen atom or
R³ and R⁴ together represent an oxygen atom,
R⁵ denotes a thienyl or naphthyl group which is optionally substituted by fluorine or chlorine,
R⁶ represents a hydrogen atom or a methyl group,
R⁷ denotes a phenyl group which is monosubstituted by amino, C₁₋₄-alkylamino or di-C₁₋₄-alkylamino, or a thienyl, naphthyl, benzofuryl, benzothienyl, indolyl, N-methylindolyl or indenyl group which is optionally substituted by fluorine, chlorine or nitro, or a C₃- to C₆-cycloalkyl group,
R⁸ denotes hydrogen, fluorine, chlorine, C₁₋₄-alkyl, methoxy or amino,
R⁹ represents hydrogen or a methyl group and
R¹⁰ represents hydrogen or a methyl group or
R⁹ and R¹⁰ together with the ring C atom represent a spirocyclopropane ring,
and salts thereof with physiologically tolerated acids.

2. N-Substituted 3-azabicyclo[3.2.0]heptane derivatives of the formula I according to claim 1 for use in combating diseases.

3. Use of the compounds of the formula I according to claim 1 for the preparation of medicaments.

4. Use of the compounds of the formula I according to claim 1 for the preparation of medicaments for the treatment of diseases of the central nervous system.

## Revendications

1. Dérivés de 3-azabicyclo[3.2.0]heptane N-substitués de formule I 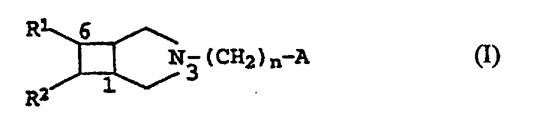 dans laquelle
R¹ représente un groupe phényle ou thiényle éventuellement mono- ou disubstitué par des atomes d'halogène ou des groupes alkyle en C₁-C₄, trifluorométhyle, hydroxy, alcoxy en C₁-C₄, amino, monométhylamino, diméthylamino, cyano ou nitro,
R² représente un atome d'hydrogène ou un groupe phényle éventuellement substitué par halogène, méthoxy, hydroxy ou amino,
n représente le nombre 1, 2, 3 ou 4,
A représente un atome d'hydrogène ou l'un des restes 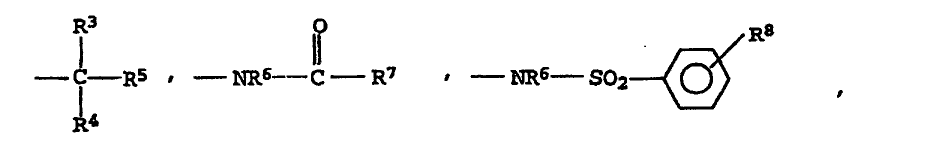 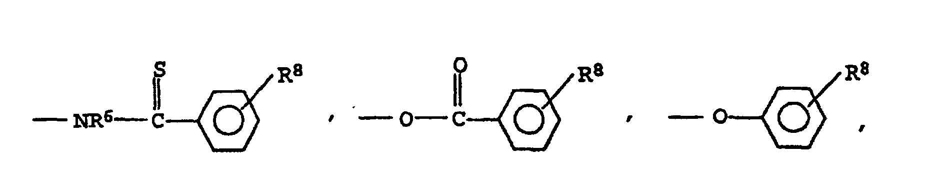 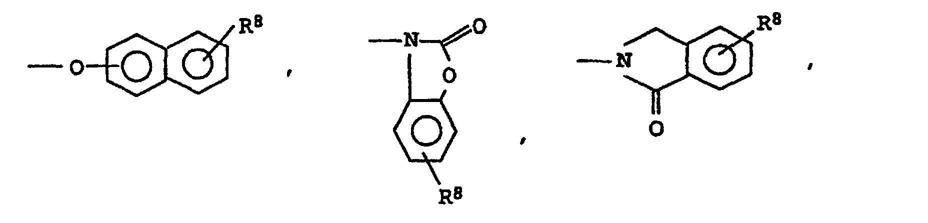 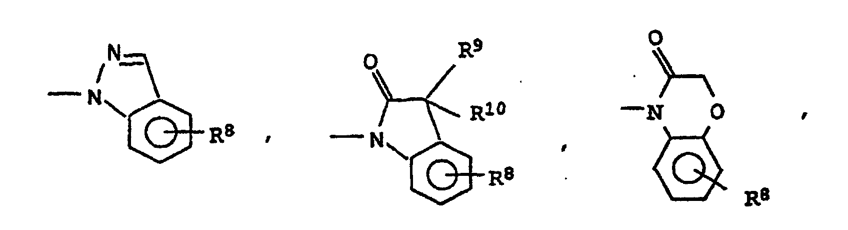 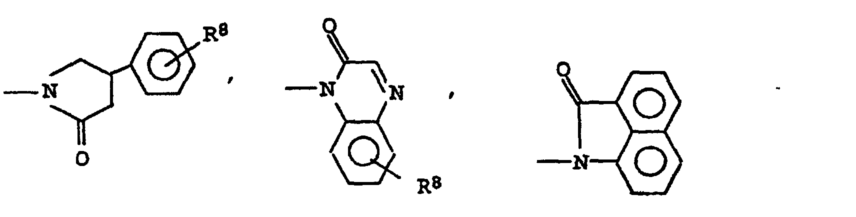 ou 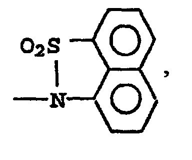
R³ représente un atome d'hydrogène ou un reste hydroxy,
R⁴ représente un atome d'hydrogène ou bien
R³ et R⁴ représentent ensemble un atome d'oxygène,
R⁵ représente un groupe thiényle ou naphtyle éventuellement substitué par du fluor ou du chlore,
R⁶ représente un atome d'hydrogène ou un groupe méthyle,
R⁷ représente un groupe phényle monosubstitué par amino, alkylamino en C₁-C₄ ou di(alkyl en C₁-C₄)amino, ou un groupe thiényle, naphtyle, benzofuryle, benzothiényle, indolyle, N-méthylindolyle ou indényle éventuellement substitué par fluoro, chloro ou nitro, ou un groupe cycloalkyle en C₃-C₆;
R⁸ représente un atome d'hydrogène, de fluor ou de chlore ou un groupe alkyle en C₁-C₄, méthoxy ou amino,
R⁹ représente un atome d'hydrogène ou un groupe méthyle et
R¹⁰ représente un atome d'hydrogène ou un groupe méthyle, ou
R⁹ et R¹⁰ représentent ensemble, avec l'atome de carbone cyclique, un cycle spirocyclopropane,
et leurs sels avec des acides physiologiquement acceptables.

2. Dérivés de 3-azabicyclo[3.2.0]heptane N-substitués de formule I selon la revendication 1, à utiliser dans la lutte contre des maladies.

3. Utilisation des composés de formule I selon la revendication 1 pour la préparation de médicaments.

4. Utilisation des composés de formule 1 selon la revendication 1 pour la préparation de médicaments destinés au traitement de maladies du système nerveux central.
